# EUROPEAN PATENT APPLICATION

(11) **EP 0 696 441 A2**
(43) Date of publication of application: **14.02.1996**
(21) Application number: 95111509.6
(22) Date of filing: 21.07.1995
(51) Int. Cl.: A61B 17/72, A61B 17/60

(54) **Means for the stabilization of long bones, especially for the osteotomy**

(30) Priority: 10.08.1994 DE 9412873 U
(71) Applicant: Howmedica GmbH, D-24232 Schönkirchen (DE)
(72) Inventor: Varlaro, Guiseppe, Dr., I-33170 Porderone (IT); Robioneck, Bernd, D-24211 Preetz (DE)
(74) Representative: Dipl.-Ing. H. Hauck, Dipl.-Ing. E. Graalfs, Dipl.-Ing. W. Wehnert, Dr.-Ing. W. Döring

(57) **Abstract**

Means for the stabilization of long bones, especially for the osteotomy, characterized by
- an externally provided fixing means comprising
- an extended supporting means and
- a plurality of pins adapted to be transversely fastened in said supporting means in different directions, said pins being led through the bones at both sides of the cut
- and a marrow area locking nail which at the ends comprises at least one through bore for receiving bone screws resp. pins of said fxing means and between the ends includes at least one elongated through slot which is passed by at least one pin of said fixing means.

## Description

The invention relates to a means for the stabilization of long bones, especially for the osteotomy, according to the preamble of the claim 1.

So-called locking nails for the stabilization of fractured long bones have become known in the most different embodiments. They are proximally or distally driven into the long bone and, in most cases, comprise a through bore for a bone screw at their ends so that at both sides of the fracture the bone fragments call be connected to said locking nail with the aid of bone nails so as to also provide a stabilization of rotation.

From the DE 39 28 460 a locking nail has become known where the distal through bore of the locking nail is defined as an elongated hole. Besides, from the DE 35 41 597 has become known to form the through bore as an elongated hole within the proximal area and to put a pressure on the bone nail with the aid of a threaded pin screwed into the proximal end so as to in this way obtain a compression. From the DE 39 21 971 a similar compression means in connection with a locking nail has become known. The hollow locking nail receives an inner part so as to be secured against torsion, said inner part being longitudinally displaceable and can be axially moved from its proximal position. It comprises a through bore which cooperates with an elongated hole in the nail. A corresponding bone screw can be led through the bore within said inner part. The described means is used for an osteotomy, said shown means allowing the edges of the osteotomy to move apart from each other by a longitudinal displacement of the inner part.

From the DE 35 37 318 a sliding slot nail has become known which proximally and distally includes slots in a cloverleaf-shaped nail profile, said nail profile comprising all open through slot of the Küntscher nail type. With the aid of such a nail it is said to be possible to drive in the locking screws without using any aiming devices and image converters.

From the EP 0 273 872, the DE 32 44 243, the DE 40 12 995 or the DE 40 12 995 locking nails have become known which, on one side only, centrically include an elongated hole or slot. The slot mostly serves the purpose of giving the nail a specific modification character.

Furthermore, it is known to externally use a so-called fixing means for the stabilization of bones. It comprises a supporting means adapted to be provided at the outside of the bone and including a plurality of adjustable pins which can be driven across the bone. Such an external fixing means can be used for the osteotomy, too. With the aid of a suitable means, a pin arranged on the supporting means can be moved in parallel so as to realize a compression or displace a segment.

It is the object of the invention to provide a means for the stabilization of long bones, especially for the osteotomy, which particularly advantageously combines a locking nail and a fixing means externally.

This problem is solved by the features of the claim 1.

According to the invention, the marrow area locking nail which at its ends is provided with through bores in a manner being known per se comprises at least one elongated through slot for receiving bone screws which slot is passed by at least one pin of said fixing means. In this way, the pin of the fxing means is externally stabilized and can move within the area of the slot only. Besides, the slot acts as a guide in case of a movement of the bone segment.

Two slots which at the adjacent ends are slightly spaced apart from each other are provided in series in an advantageous manner. Thus, the weakening of the locking nail which automatically occurs as a result of such an elongated through slot in the center area is slightly reduced. Preferably, the slots are provided on approximately the same level as the through bores for the bone nails.

According to a further embodiment of the invention, the locking nail in the usual way is a hollow body. It includes a circumferentially extending through wall and comprises a cloverleaf-shape profile in its cross-section. The clover-leaf-shape profile gives the locking nail a very high geometrical moment of inertia which means that it has a high transverse stability and/or stability of rotation. The weakening caused by the slot, thus, can be compensated again. Preferably, a cylindrical tube is used as the basic material for the locking nail which at circumferential distances of 120° is radially pressed inwardly so as to get the cloverleaf-shape profile.

The invention will be more detailedly explained hereinafter with the aid of drawings.
- Fig. 1: shows a perspective view of the combination of an externally provided fixing means comprising a locking nail according to the invention after implantation of the nail and the proximal osteotomy.
- Fig. 2: shows a side view of the locking nail according to the Figures 1 and 2.
- Fig. 3: shows a view of the nail according to Fig. 2 towards arrow 4.
- Fig. 4: shows a section through the nail according to Fig. 3 taken along line 4-4.

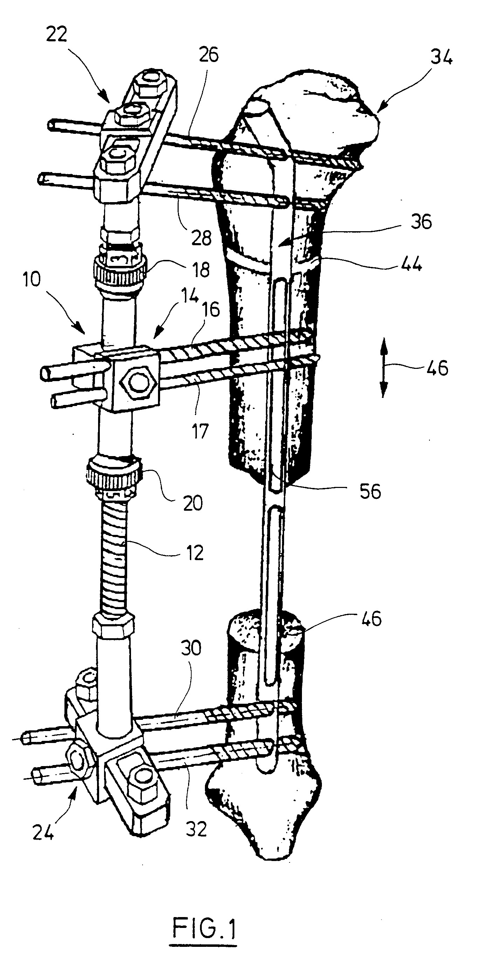

The fixing means 10 according to Fig. 1 comprises a threaded spindle 12 where a first supporting means 14 for two bone pins 16, 17 is displaceably arranged on. They are displaced by moving the knurled nuts 18 resp. 20. The displacement is shown by a double arrow 46 in Fig. 1. At the ends of the spindle 12 a second resp. third supporting means 22, 24 is arranged for two bone pins 26, 28, 30 and 32. The fixing of the bone pins 16, 17 resp. 26 to 32 is not described more detailedly since it is generally known. The pins can be fixed in any restricted angular position whatever.

A locking nail 36 is driven into the shown tibia 34 from a proximal position which locking nail at the proximal as well as at the distal end receives transverse through bores for receiving bone screws or the threaded pins 26 to 32 as shown. The insertion of the threaded pins makes the use of a suitable aiming device necessary which often includes an image intensifier. This, however, is not further discussed as it is already generally known.

As can be seen furthermore, the bone pin 16, 17 extends through a longitudinal slot 46 of the locking nail 36.

Fig. 1 shows that a bone segment 42 has been separated by a cut as shown at 44 resp. 46. Furthermore, the bone 34 had been distracted so as to form a recess. Thereafter, as a result of the described displacement of the supporting means 14, the bone segment 42 is moved downwardly as arrow 46 shows. This movement causes the bone pins 16, 17 to move within the area of the slot of the locking nail 36. The free space above the segment 42 e.g. is filled up with bone substance or suchlike.

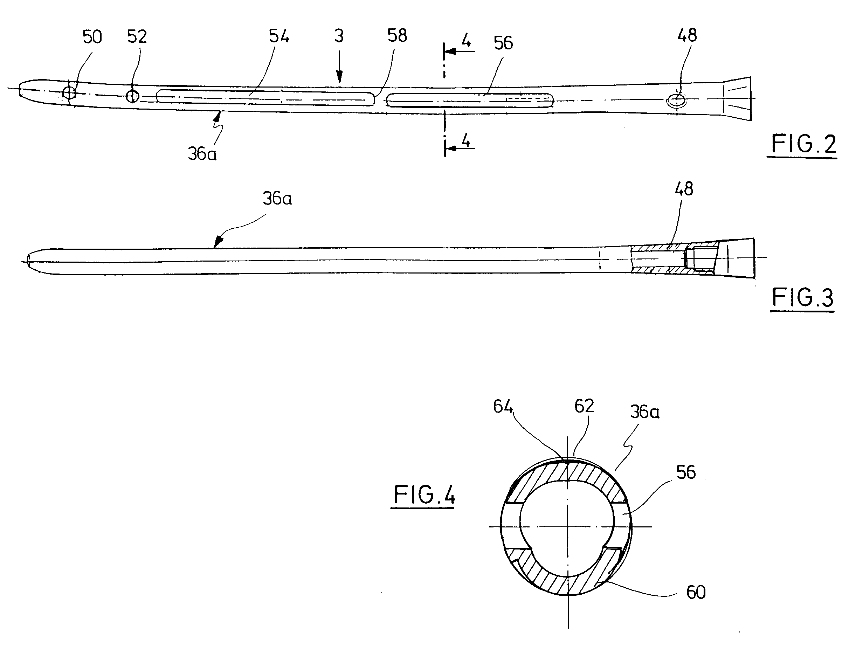

Figs. 2 and 4 show a locking nail for the femur which, however, with respect to its construction, is similar to the locking nail according to Fig. 1. It, therefore, has been provided with the reference number 36a. The hollow nail 36a at the proximal end comprises an inclined through bore 48 and at the distal end includes two circular spaced through bores 50, 52 for the threaded pins mentioned in connection with Fig. 1. Moreover, in the center area the shaft comprises two longitudinal slots 54, 56 which are separated from each other by a relatively narrow space 58. The lengths over which both slots 54, 56 extend approximately correspond to half the length of the complete nail 36a, with the distance between the distal end of the slot 54 and the distal nail end being smaller than the distance between the proximal end of the slot 56 and the proximal nail end. The width of the slots 54, 56 is slightly larger than the diameter of the bores 50, 52.

As appears from Fig. 4, the hollow nail 36a includes a through wall 60 all around. The nail is first formed of a cylindrical tube as shown by the dash-dot line 62. It then is radially pressed inwardly in positions being offset by 120° as shown at 64 so as to get a clover-leaf-shape profile which increases the stability of the nail 36a in transverse and circumferential direction.

## Claims

1. Means for the stabilization of long bones, especially for the osteotomy, characterized by
- an externally provided fixing means (10) comprising
- an extended supporting means (12) and
- a plurality of pins (16, 17, 26, 28, 30, 32) adapted to be transversely fastened in said supporting means in different directions, said pins being led through the bones at both sides of the cut
- and a marrow area locking nail (36, 36a) which at the ends comprises at least one through bore (50, 52, 48) for receiving bone screws resp. pins of said fixing means (10) and between the ends includes at least one elongated through slot (54, 56) which is passed by at least one pin (16, 17) of said fixing means (10).

2. The means according to claim 1, characterized in that two slots (54, 56) arranged in series are provided which at the adjacent ends are separated from each other by a small space (58).

3. The means according to claim 1 or 2,characterized in that the axes of said through bores (50, 52, 48) and said slot (54, 56) extend parallel to each other.

4. The means according to any of the claims 1 to 3, characterized in that said locking nail (36, 36a) is a hollow body which comprises a circumferentially extending through wall (60) and a cloverleaf-shape profile in its cross-section.

5. The means according to claim 4, characterized in that said locking nail (36a) is formed of a cylindrical tube (62) which is radially pressed inwardly (64) at circumferential distances of 120°.

6. The means according to any of the claims 1 to 5, characterized in that at least two pins (16, 17) of said fixing means (10) are led through a longitudinal slot (56).
